# EUROPÄISCHE PATENTANMELDUNG

(11) **EP 1 104 810 A1**
(43) Veröffentlichungstag der Anmeldung: **06.06.2001**
(21) Anmeldenummer: 00122575.4
(22) Anmeldetag: 17.10.2000
(51) Int. Cl.: C12N 15/31, C12P 13/04, C12P 13/08, C07K 14/34

(54) **Verfahren zur fermentativen Herstellung von L-Lysin unter Verwendung coryneformer Bakterien**

(30) Priorität: 09.11.1999 DE 19953809
(71) Anmelder: Degussa AG, 40474 Düsseldorf (DE)
(72) Erfinder: Möckel, Bettina, Dr., 40597 Düsseldorf (DE); Pfefferle, Walter, Dr., 33790 Halle (Westf.) (DE); Brand, Sven, 33613 Bielefeld (DE); Pühler, Alfred, Prof., 33739 Bielefeld (DE); Kalinowski, Jörn, Dr., 33615 Bielefeld (DE); Bathe, Brigitte, Dr., 33154 Salzkotten (DE)

(57) **Zusammenfassung**

Die Erfindung betrifft ein Verfahren zur Herstellung von L-Aminosäuren, insbesondere L-Lysin, bei dem man folgende Schritte durchführt,
7.
a) Fermentation der die gewünschte L-Aminosäure produzierenden Bakterien, in denen man zumindest das csp1-Gen abschwächt,
b) Anreicherung des gewünschten Produkts im Medium oder in den Zellen der Bakterien, und
c) Isolieren der L-Aminosäure,
und gegebenenfalls Bakterien einsetzt, in denen man zusätzlich weitere Gene des Biosyntheseweges der gewünschten L-Aminosäure verstärkt, oder Bakterien einsetzt, in denen die Stoffwechselwege zumindest teilweise ausgeschaltet sind, die die Bildung der gewünschten L-Aminosäure verringern.

## Beschreibung

Gegenstand der Erfindung ist ein Verfahren zur fermentativen Herstellung von L-Aminosäuren, insbesondere L-Lysin, unter Verwendung coryneformer Bakterien, in denen das cspl-Gen abgeschwächt ist.

### Stand der Technik

L-Aminosäuren, insbesondere L-Lysin finden in der Tierernährung, in der Humanmedizin und in der pharmazeutischen Industrie Anwendung.

Es ist bekannt, daß diese Aminosäuren durch Fermentation von Stämmen coryneformer Bakterien, insbesondere Corynebacterium glutamicum hergestellt werden. Wegen der großen Bedeutung wird ständig an der Verbesserung der Herstellverfahren gearbeitet. Verfahrensbesserungen können fermentationstechnische Maßnahmen wie z. B. Rührung und Versorgung mit Sauerstoff, oder die Zusammensetzung der Nährmedien wie z. B. die Zuckerkonzentration während der Fermentation, oder die Aufarbeitung zur Produktform durch z. B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z. B. das Lysin-Analogon S-(2-Aminoethyl)-Cystein oder auxotroph für regulatorisch bedeutsame Metabolite sind und L-Aminosäuren produzieren.

Seit einigen Jahren werden ebenfalls Methoden der rekombinanten DNA-Technik zur Stammverbesserung L-Aminosäure produzierender Stämme von Corynebacterium glutamicum eingesetzt, indem man einzelne Aminosäure-Biosynthesegene amplifiziert und die Auswirkung auf die L-Aminosäure-Produktion untersucht. Übersichtsartikel hierzu findet man unter anderem bei Kinoshita (,,Glutamic Acid Bacteria,,, in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6, 261-272 (1994)), Jetten und Sinskey (Critical Reviews in Biotechnology 15, 73-103 (1995)) und Sahm et al. (Annuals of the New York Academy of Science 782, 25-39 (1996)).

### Aufgabe der Erfindung

Die Erfinder haben sich die Aufgabe gestellt, neue Grundlagen für verbesserte Verfahren zur fermentativen Herstellung von L-Aminosäuren, insbesondere L-Lysin, mit coryneformen Bakterien bereitzustellen.

### Beschreibung der Erfindung

L-Aminosäuren, insbesondere Lysin, finden in der Humanmedizin und in der pharmazeutischen Industrie, in der Lebensmittelindustrie und ganz besonders in der Tierernährung Anwendung. Es besteht daher ein allgemeines Interesse daran, neue verbesserte Verfahren zur Herstellung von Aminosäuren, insbesondere L-Lysin, bereitzustellen.

Wenn im Folgenden L-Lysin oder Lysin erwähnt werden, ist damit nicht nur die Base, sondern es sind auch die Salze wie z. B. Lysin-Monohydrochlorid oder Lysin-Sulfat gemeint.

Gegenstand der Erfindung ist ein Verfahren zur fermentativen Herstellung von L-Aminosäuren, insbesondere L-Lysin, unter Verwendung von coryneformen Bakterien, in denen man zumindest die für das Csp1-Genprodukt kodierende Nucleotidsequenz (csp1-Gen) abschwächt, insbesondere auf niedrigem Niveau exprimiert, das gewünschte Produkt im Medium oder in den Zellen anreichert und die L-Aminosäure isoliert.

Die eingesetzten Stämme produzieren bevorzugt bereits vor der Abschwächung des csp1-Gens L-Aminosäuren, insbesondere L-Lysin.

Bevorzugte Ausführungsformen finden sich in den Ansprüchen.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA (hier csp1-Gen) kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Die Mikroorganismen, die Gegenstand der vorliegenden Erfindung sind, können Aminosäuren, insbesondere Lysin, aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind besonders die bekannten Wildtypstämme
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme
wie beispielsweise die L-Lysin produzierenden Stämme
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM 5714

Es wurde gefunden, daß coryneforme Bakterien nach Abschwächung des csp1-Gens in verbesserter Weise L-Aminosäuren, insbesondere L-Lysin, produzieren.

Das csp1-Gen codiert für das PS1-Protein, für das bislang keine enzymatische Aktivität nachgewiesen werden konnte. Die Nukleotidsequenz des csp1-Gens wurde von Joliff et al. (Molecular Microbiology 1992 Aug; 6 (16):2349-62) beschrieben. Sie ist bei der Nukleotidsequenzdaten-Bank des National Center for Biotechnology Information (NCBI, Bethesda, MD, USA) unter der accession number g40486 allgemein verfügbar. Das in den angegebenen Textstellen beschriebene csp1-Gen kann erfindungsgemäß verwendet werden. Weiterhin können Allele des csp1-Gens verwendet werden, die sich aus der Degeneriertheit des genetischen Codes oder durch funktionsneutrale Sinnmutationen (sense mutations) ergeben.

Zur Erzielung einer Abschwächung können entweder die Expression des csp1-Gens oder die katalytischen Eigenschaften des Genproduktes herabgesetzt oder ausgeschaltet werden. Gegebenenfalls werden beide Maßnahmen kombiniert.

Die Genexpression kann durch geeignete Kulturführung oder durch genetische Veränderung (Mutation) der Signalstrukturen der Genexpression verringert werden. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z. B. in der Patentanmeldung WO 96/15246, bei Boyd und Murphy (Journal of Bacteriology 170: 5949 (1988)), bei Voskuil und Chambliss (Nucleic Acids Research 26: 3548 (1998), bei Jensen und Hammer (Biotechnology and Bioengineering 58: 191 (1998)), bei Pátek et al. (Microbiology 142: 1297 (1996)) und in bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung bzw. Herabsetzung der katalytischen Eigenschaften von Enzymproteinen führen, sind aus dem Stand der Technik bekannt; als Beispiele seien die Arbeiten von Qiu und Goodman (Journal of Biological Chemistry 272: 8611-8617 (1997)), Sugimoto et al. (Bioscience Biotechnology and Biochemistry 61: 1760-1762 (1997)) und Möckel ("Die Threonindehydratase aus Corynebacterium glutamicum: Aufhebung der allosterischen Regulation und Struktur des Enzyms", Berichte des Forschungszentrums Jülichs,Jül-2906, ISSN09442952, Jülich, Deutschland, 1994) genannt. Zusammenfassende Darstellungen können bekannten Lehrbüchern der Genetik und Molekularbiologie wie z. B. dem von Hagemann ("Allgemeine Genetik", Gustav Fischer Verlag, Stuttgart, 1986) entnommen werden.

Als Mutationen kommen Transitionen, Transversionen, Insertionen und Deletionen in Betracht. In Abhängigkeit von der Wirkung des Aminosäureaustausches auf die Enzymaktivität wird von Fehlsinnmutationen (missense mutations) oder Nichtsinnmutationen (nonsense mutations) gesprochen. Insertionen oder Deletionen von mindestens einem Basenpaar in einem Gen führen zu Rasterverschiebungsmutationen (frame shift mutations), in deren Folge falsche Aminosäuren eingebaut werden oder die Translation vorzeitig abbricht. Deletionen von mehreren Kodonen führen typischerweise zu einem vollständigen Ausfall der Enzymaktivität. Anleitungen zur Erzeugung derartiger Mutationen gehören zum Stand der Technik und können bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

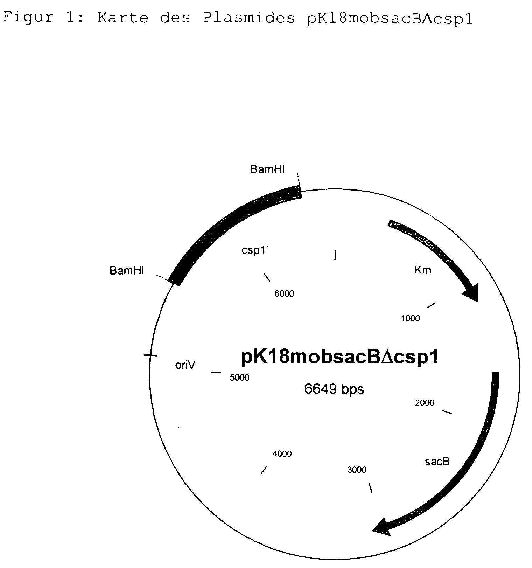

Ein Beispiel für ein mutiertes csp1-Gen ist das in Plasmid pK18mobsacBΔcspl (Figur 1) enthaltene Δcsp1-Allel. Das Δcsp1-Allel enthält lediglich Sequenzen der 5'- und der 3'-Flanke des csp1-Gens; ein 1690 bp langer Abschnitt der Kodierregion fehlt (Deletion). Dieses Δcsp-Alle1 kann durch Integrationsmutagenese in coryneforme Bakterien eingebaut werden. Hierzu bedient man sich des oben angegebenen Plasmides pK18mobsacBΔcsp1, das in C. glutamicum nicht replizierbar ist. Nach Transformation und homologer Rekombination mittels eines ersten, Integration bewirkenden "cross over"-Ereignisses und eines zweiten, eine Excision bewirkenden "cross over"-Ereignisses im csp1-Gen erreicht man den Einbau der Δcsp1-Deletion und erzielt einen Totalverlust der Funktion in dem jeweiligen Stamm.

Anleitungen und Erläuterungen zur Integrationsmutagenese findet man beispielsweise bei Schwarzer und Pühler (Bio/Technology 9,84-87 (1991)) oder Peters-Wendisch et al. (Microbiology 144, 915-927 (1998)).

Ein Beispiel für einen Aminosäure produzierenden Stamm coryneformer Bakterien mit abgeschwächtem csp1-Gen ist der Lysinproduzent Corynebacterium glutamicum R167Δcsp1.

Zusätzlich kann es für die Produktion von Aminosäuren, insbesondere L-Lysin vorteilhaft sein, zusätzlich zur Abschwächung des csp1-Gens, eines oder mehrere Enzyme des jeweiligen Biosyntheseweges, der Glykolyse, der Anaplerotik, des Zitronensäure-Zyklus oder des Aminosäure-Exports zu verstärken.

So kann beispielsweise für die Herstellung von L-Lysin
- gleichzeitig das für die Dihydrodipicolinat-Synthase kodierende dapA-Gen (EP-B 0 197 335), und/oder
- gleichzeitig das für die Glyceraldehyd-3-Phosphat Dehydrogenase kodierende gap-Gen (Eikmanns (1992). Journal of Bacteriology 174:6076-6086), oder
- gleichzeitig das für die Pyruvat Carboxylase codierende pyc-Gen(Eikmanns (1992). Journal of Bacteriology 174:6076-6086), oder
- gleichzeitig das für die Malat:Chinon Oxidoreduktase kodierende mqo-Gen (Molenaar et al., European Journal of Biochemistry 254, 395 - 403 (1998)), oder
- gleichzeitig das für den Lysin-Export kodierende lysE-Gen (DE-A-195 48 222)
überexprimiert werden.

Weiterhin kann es für die Produktion von Aminosäuren, insbesondere L-Lysin vorteilhaft sein, neben dem csp1-Gen gleichzeitig
- das für die Phosphoenolpyruvat-Carboxykinase codierende pck-Gen (DE 199 50 409.1, DSM 13047) und/oder
- das für die Glucose-6-Phosphat Isomerase kodierende pgi-Gen (US 09/396,478, DSM 12969)
abzuschwächen.

Schließlich kann es für die Produktion von Aminosäuren, insbesondere L-Lysin, vorteilhaft sein, neben der Abschwächung des cspl-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnußöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoffhaltige Gasmischungen wie z. B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann so wie bei Spackman et al. (Analytical Chemistry, 30, (1958), 1190) beschrieben durch Anionenaustauschchromatographie mit anschließender Ninhydrin Derivatisierung erfolgen, oder sie kann durch reversed phase HPLC erfolgen, so wie bei Lindroth et al. (Analytical Chemistry (1979) 51: 1167-1174) beschrieben.

Folgender Mikroorganismus wurde bei der Deutschen Sammlung für Mikroorganismen und Zellkulturen (DSMZ, Braunschweig, Deutschland) gemäß Budapester Vertrag hinterlegt:
- Escherichia coli Stamm S17-1 /pK18mobsacBΔcsp1 als DSM 13048

### Beispiele

Die vorliegende Erfindung wird im Folgenden anhand von Ausführungsbeispielen näher erläutert.

### Beispiel 1

### Herstellung eines Deletionsvektors für die Deletionsmutagenese des csp1-Gens

Aus dem Stamm ATCC 13032 wurde nach der Methode von Eikmanns et al. (Microbiology 140:1817-1828 (1994)) chromosomale DNA isoliert. Die Nukleotidsequenz des cspl-Gens für C. glutamicum ist bei der Nukleotidsequenzdaten-Bank des National Center for Biotechnology Information (NCBI, Bethesda, MD, USA) unter der accession number g40486 verfügbar. Aufgrund der bekannten Sequenz wurden die folgenden Oligonukleotide für die Polymerase Kettenreaktion ausgewählt:
csp1-10:
5' GAT CTA G(GA TC)C CGA TGA GCG CGT CCA TGT GT 3' csp1-11:
5' GAT CTA G(GA TC)C TCG ACC TTG CGG TGC TGC TT 3'
csp1-del:
5' GGA ATA CGT AGC CAC CTT CGG TCC CGA AAG TTC CCC GCT T 3'

Die dargestellten Primer wurden von der Firma MWG Biotech (Ebersberg, Deutschland) synthetisiert und nach der PCR-Methode von Karreman (BioTechniques 24:736-742, 1998) mit Pwo-Polymerase der Firma Boehringer die PCR Reaktion durchgeführt. Die Primer cspl-10 und csp1-11 enthalten eingefügt jeweils eine Schnittstelle für das Restriktionenzym BamHI, die in der Darstellung in Klammern angegeben sind. Mit Hilfe der Polymerase-Kettenreaktion wurde ein ca. 0,9 kb großes DNA-Fragment amplifiziert und isoliert, welches eine 1690 bp große Deletion des csp1-Gens trägt.

Das amplifizierte DNA Fragment wurde mit dem Restriktionsenzym BamHI geschnitten und aus einem Agarosegel (0,8% aufgereinigt). Ebenso wurde das Plasmid pK18mobsacB (Jäger et al., Journal of Bacteriology, 1:784-791 (1992)) mit dem Restriktionsenzym BamHI geschnitten. Das Plasmid pK18mobsacB und das PCR-Fragment wurden ligiert. Anschließend wurde der E. coli Stamm S17-1 (Simon et al., 1993, Bio/Technology 1:784-791) mit dem Ligationsansatz (Hanahan, In: DNA cloning. A practical approach. Vol.I. IRL-Press, Oxford, Washington DC, USA, 1985) elektroporiert. Die Selektion von Plasmid-tragenden Zellen erfolgte durch Ausplattieren des Transformationsansatzes auf LB Agar (Sambrook et al., Molecular cloning: a laboratory manual. 2^{nd} Ed. Cold Spring Harbor Laboratory Press, Cold Spring Harbor, N.Y., 1989), der mit 25 mg/l Kanamycin supplementiert worden war. Plasmid-DNA wurde aus einer Transformante mit Hilfe des QIAprep Spin Miniprep Kit der Firma Qiagen isoliert und durch Restriktion mit dem Restriktionsenzym BamHI und anschließender Agarosegel-Elektrophorese (0,8%) überprüft. Das Plasmid wurde pK18mobsacBΔcsp1 genannt. Der Stamm wurde als E. coli S17-1 /pK18mobsacBΔcsp1 bezeichnet und ist unter der Nummer DSM 13048 bei der Deutschen Sammlung für Mikroorganismen und Zellkulturen (DSMZ, Braunschweig, Deutschland) hinterlegt.

### Beispiel 2

### Deletionsmutagenese des cspl-Gens in dem C. glutamicum Wildtyp R167

Der in Beispiel 2 genannte Vektor pK18mobsacBΔcsp1 wurde nach der Elektroporationsmethode von Tauch et al.(FEMS Microbiological Letters, 123:343-347 (1994)) in Corynebacterium glutamicum R167 (Liebl et al. (1989) FEMS Microbiological Letters 65:299-304) elektroporiert. Bei dem Stamm R167 handelt es sich um einen restriktionsdefizienten C. glutamicum Wildtypstamm. Der Vektor pK18mobsacBΔcsp1 kann in C. glutamicum nicht selbständig replizieren und bleibt nur dann in der Zelle erhalten, wenn er ins Chromosom integriert hat. Die Selektion von Klonen mit integriertem pK18mobsacBΔcsp1 erfolgte durch Ausplattieren des Elektroporationsansatzes auf LB Agar (Sambrook et al., Molecular cloning: a laboratory manual. 2^{nd} Ed. Cold Spring Harbor Laboratory Press, Cold Spring Harbor, N.Y., 1989), der mit 15 mg/l Kanamycin supplementiert worden war. Angewachsene Klone wurden auf LB-Agarplatten mit 25 mg/l Kanamycin ausgestrichen und für 16 Stunden bei 33°C inkubiert. Um die Excision des Plasmides zusammen mit der vollständigen chromosomalen Kopie des csp1-Gens zu erreichen, wurden die Klone anschließend auf LB-Agar mit 10% Sucrose angezogen. Das Plasmid pK18mobsacB enthält eine Kopie des sacB-Gens, welches Sucrose in die für C. glutamicum toxische Levansucrase umwandelt. Auf LB-Agar mit Sucrose wachsen daher nur solche Klone an, bei denen das integrierte pK18mobsacBΔcsp1 wiederum excisiert hat. Bei der Excision kann zusammen mit dem Plasmid entweder die vollständige chromosomale Kopie des csp1-Gens excisieren, oder die unvollständige Kopie mit der internen Deletion. Um nachzuweisen, daß die unvollständige Kopie von csp1 im Chromosom verblieben ist, wurde das Plasmid pK18mobsacBΔcsp1 Fragment nach der Methode "The DIG System Users Guide for Filter Hybridization" der Firma Boehringer Mannheim GmbH (Mannheim, Deutschland, 1993) mit dem Dig-Hybridisierungskit der Firma Boehringer markiert. Chromosomale DNA einer potentiellen Deletionsmutante wurde nach der Methode von Eikmanns et al. (Microbiology 140: 1817-1828 (1994)) isoliert und jeweils mit dem Restriktionsenzymen EcoRI geschnitten. Die entstehenden Fragmente wurden mit Agarosegel-Elektrophorese aufgetrennt und mit dem Dig-Hybrisierungskit der Firma Boehringer bei 68°C hybridisiert. Bei dem Kontrollstamm wurden zwei hybridisierende Fragmente von ca. 6500 bp und ca. 4000 bp erhalten, während bei der Mutante zwei hybridisierende Fragmente von ca. 6500 bp und ca. 3200 bp erhalten wurden. Damit konnte gezeigt werden, daß der Stamm R167 seine vollständige Kopie des csp1-Gens verloren hat und stattdessen nur noch über die unvollständige Kopie mit der Deletion von ca. 1690 bp verfügt. Der Stamm wurde als C. glutamicum R167Δcsp1 bezeichnet.

### Beispiel 3

### Herstellung von Lysin

Der in Beispiel 2 erhaltene C. glutamicum Stamm R167Δcsp1 wurde in einem zur Produktion von Lysin geeigneten Nährmedium kultiviert und der Lysingehalt im Kulturüberstand bestimmt.

Dazu wurde der Stamm zunächst auf Agarplatte für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet. Die Vorkultur wurde 48 Stunden bei 33°C bei 240 rpm auf dem Schüttler inkubiert. Von dieser Vorkultur wurde eine Hauptkultur angeimpft, so daß die Anfangs-OD (660 nm) der Hauptkultur 0,1 OD betrug. Für die Hauptkultur wurde das Medium MM verwendet.

| | |
|---|---|
| Medium MM | |
| CSL (Corn Steep Liquor) | 5 g/l |
| MOPS | 20 g/l |
| Glucose (getrennt autoklaviert) | 50g/l |
| Salze: | |
| (NH₄)₂SO₄) | 25 g/1 |
| KH2PO₄ | 0,1 g/l |
| MgSO₄ ∗ 7 H₂O | 1,0 g/l |
| CaCl₂ ∗ 2 H₂O | 10 mg/l |
| FeSO₄ ∗ 7 H₂O | 10 mg/l |
| MnSO₄ ∗ H₂O | 5, 0mg/l |
| Biotin (sterilfiltriert) | 0,3 mg/l |
| Thiamin ∗ HCl (sterilfiltriert) | 0,2 mg/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃ zugesetzt.

Die Kultivierung erfolgte in 10 ml Volumen in einem 100 ml Erlenmeyerkolben mit Schikanen. Die Kultivierung erfolgte bei 33°C und 80% Luftfeuchte.

Nach 48 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD(660) | Lysin-HCl g/l |
|---|---|---|
| R167 | 13,8 | 0,00 |
| R167Δcsp1 | 12,6 | 0,99 |

Folgende Figuren sind beigefügt:
Figur 1: Karte des Plasmids pK18mobsacBΔcsp1.

Die verwendeten Abkürzungen und Bezeichnungen haben folgende Bedeutung. Längenangaben sind als ca.-Werte aufzufassen.
- sacB:: sacB-Gen
- oriV:: Replikationsursprung V
- KmR:: Kanamycin Resistenz
- BamHI:: Schnittstelle des Restriktionsenzyms BamHI
- csp1':: unvollständiges Fragment des csp1-Gens mit interner 1690 bp Deletion

## Patentansprüche

1. Verfahren zur Herstellung von L-Aminosäuren, insbesondere L-Lysin,
**dadurch gekennzeichnet,**
daß man folgende Schritte durchführt,
a) Fermentation der die gewünschte L-Aminosäure produzierenden Bakterien, in denen man zumindest das csp1-Gen abschwächt,
b) Anreicherung des gewünschten Produkts im Medium oder in den Zellen der Bakterien, und
c) Isolieren der L-Aminosäure.

2. Verfahren gemäß Anspruch 1,
**dadurch gekennzeichnet,**
daß man Bakterien einsetzt, in denen man zusätzlich weitere Gene des Biosyntheseweges der gewünschten L-Aminosäure verstärkt.

3. Verfahren gemäß Anspruch 1,
**dadurch gekennzeichnet,**
daß man Bakterien einsetzt, in denen die Stoffwechselwege zumindest teilweise ausgeschaltet sind, die die Bildung der gewünschten L-Aminosäure verringern.

4. Verfahren gemäß Anspruch 1,
**dadurch gekennzeichnet,**
daß man die Expression des Polynukleotids, das für das cspl-Gen codiert, verringert.

5. Verfahren gemäß Anspruch 1,
**dadurch gekennzeichnet,**
daß man die katalytischen Eigenschaften des Polypeptids (Enzymproteins) herabsetzt, für das das Polynukleotid cspl codiert.

6. Verfahren gemäß Anspruch 1,
**dadurch gekennzeichnet,**
daß man zur Erzielung der Abschwächung das Verfahren der Integrationsmutagenese mittels des Vektors pK18mobsacBΔcsp1, dargestellt in Figur 1 und hinterlegt in E.coli als DSM 13048, verwendet.

7. Verfahren gemäß Anspruch 1,
**dadurch gekennzeichnet,**
daß man für die Herstellung von L-Lysin Bakterien fermentiert, in denen man gleichzeitig eines oder mehrere der Gene, ausgewählt aus der Gruppe
7.1 das für die Dihydrodipicolinat-Synthase kodierende dapA-Gen,
7.2 ein S-(2-Aminoethyl)-Cystein-Resistenz vermittelndes DNA-Fragment,
7.3 das für die Pyruvat-Carboxylase kodierende pyc-Gen,
7.4 das für die Tetradihydrodipicolinat Succinylase kodierende dapD-Gen,
7.5 das Gen für die Succinyldiaminopimelate-Desuccinylase kodierende dapE-Gen,
7.6 das für die Glyceraldehyd-3-Phosphat Dehydrogenase kodierende gap-Gen,
7.7 das für die Malat:Chinon Oxidoreduktase kodierende mqo-Gen,
7.8 das für den Lysin-Export kodierende lysE-Gen, gleichzeitig überexprimiert oder amplifiziert.

8. Verfahren gemäß Anspruch 1,
**dadurch gekennzeichnet,**
daß man für die Herstellung von L-Lysin Bakterien fermentiert, in denen man gleichzeitig eines oder mehrere der Gene, ausgewählt aus der Gruppe
8.1 das für die Phosphoenolpyruvat-Carboxykinase codierende pck-Gen,
8.2 das für die Glucose-6-Phosphat Isomerase kodierende pgi-Gen
abschwächt.

9. Verfahren gemäß einem oder mehreren der vorhergehenden Ansprüche,
**dadurch gekennzeichnet,**
daß man Mikroorganismen der Gattung Corynebacterium glutamicum einsetzt.
